# EUROPÄISCHE PATENTSCHRIFT

(11) **EP 2 807 999 B1**
(45) Veröffentlichungstag und Bekanntmachung des Hinweises auf die Patenterteilung: **27.05.2015**
(21) Anmeldenummer: 13169423.4
(22) Anmeldetag: 28.05.2013
(51) Int. Cl.: A61F 13/62, A44B 18/00

(54) **Verbundstoffbahn mit elastischen und unelastischen Bereichen, Verfahren zur Herstellung einer Verbundstoffbahn sowie Hakenmaterial in Streifenform, insbesondere für eine Verbundstoffbahn**
Composite fabric sheet with inelastic and elastic regions, method for producing the same and hook material in strip form, in particular for a composite fabric sheet
Bande de matériau composite dotée de zones élastiques et non élastiques, procédé de fabrication d'une bande de matériau composite et matériau de crochet en forme de bande, notamment pour une bande de matériau composite

(43) Veröffentlichungstag der Anmeldung: 03.12.2014
(60) Teilanmeldung: 15158574.2
(73) Patentinhaber: Mondi Consumer Packaging Technologies GmbH, 48599 Gronau (DE)
(72) Erfinder: Bader, Herbert, 48356 Nordwalde (DE); Kurrer, Horst, 48599 Gronau (DE); Grossmann, Marcel, 45309 Essen (DE); Fezert, Olga, 49809 Lingen (DE)
(74) Vertreter: Lorenz, Bernd Ingo Thaddeus

(56) Entgegenhaltungen:
- EP-A1- 2 301 502
- WO-A1-99/13745
- US-A- 6 063 466

## Beschreibung

Die Erfindung betrifft eine Verbundstoffbahn mit elastischen und unelastischen Bereichen, aus der Windelverschlusselemente ausstanzbar sind. Die Verbundstoffbahn umfasst eine Nonwovenbahn, welche eine erste Außenseite der Verbundstoffbahn bildet, zueinander beabstandete, üblicherweise parallel angeordnete elastisch dehnbare Folienstreifen, die auf der Nonwovenbahn angeordnet sind, ein Nonwomenmaterial, welches an einer zweiten Außenseite der Verbundstoffbahn die Folienstreifen abdeckt und zumindest einen nichtelastischen Streifen aus einem Hakenmaterial, der einen Bereich zwischen zwei benachbarten elastischen dehnbaren Folienstreifen überbrückt.

Bei der Herstellung von Verbundstoffbahnen für Windelverschlusselemente ist es aus der EP 2 301 502 A1 bekannt, elastische Streifen zwischen zwei flächige Nonwovenbahnen einzukaschieren, wobei zwischen zwei benachbarten elastisch dehnbaren Folienstreifen wechselweise eine direkte Verbindung der beiden Nonwovenbahnen oder die Einbindung eines Verstärkungsstreifens erfolgt. Dort, wo die beiden Nonwovenbahnen direkt miteinander verbunden sind, kann das Windelverschlusselement an einem Wegwerfartikel wie einer Babywindel oder einem Hygieneprodukt für Erwachsene befestigt werden, während der Abschnitt mit dem elastisch dehnbaren Folienstreifen dem Windelverschlusselement die erforderliche Elastizität verleiht. Dort, wo der Verstärkungsstreifen angeordnet ist, kann dann nachträglich eine Hakenband aufgebracht werden, wobei der Verstärkungsstreifen dazu notwendig ist, dass das Nonwovenmaterial unter Zug nicht unkontrolliert zerreißt. Aus dem gleichen Grund ist auch eine Überlappung zwischen dem Verstärkungsstreifen und dem elastisch dehnbaren Folienstreifen vorgesehen.

Eine Verbundstoffbahn mit elastischen und unelastischen Bereichen ist auch aus der EP 1 736 306 A1 bekannt, wobei dort elastische und unelastische Bereiche durch einen entsprechenden Klebstoffauftrag gebildet werden. Der Klebstoff ist dabei dazu vorgesehen, das Material in ausreichendem Maße zu verfestigen, wobei entsprechend an elastisch dehnbaren Folienstreifen nur eine abschnittsweise Verklebung vorgesehen ist. Die nachträgliche Anordnung eines Hakenmaterials an der Außenseite kann jedoch problematisch sein, weil das Material unterhalb des Hakenbandes nur durch eine durchgehende Klebstoffschicht verstärkt ist.

Eine Verbundstoffbahn mit den eingangs beschriebenen Merkmalen ist aus der EP 2 340 796 A1 bekannt, wobei auch hier das Hakenmaterial außen auf eine von zwei flächigen Nonwovenbahnen aufgeklebt ist. Um dem Material insgesamt eine ausreichende Festigkeit zu verleihen, ist an der gegenüberliegenden Seite ein weiterer, nichtelastischer Streifen zur Verstärkung vorgesehen. Die Applikation von zwei gegenüberliegenden nichtelastischen Materialstreifen ist hinsichtlich der Materialkosten und der Verfahrensführung aufwendig.

Aus der EP 0 768 075 B1 ist eine Wegwerfwindel mit seitlichen Wiederverschlusselementen bekannt, die keine elastischen Bereiche aufweisen. Die notwendige Elastizität der gesamten Wegwerfwindel wird dadurch erreicht, dass in einem vorderen Taillenbereich ein elastisches Element angeordnet ist, während die Windelverschlusselemente in Form von seitlichen Flügeln starr sind. An den seitlichen Windelverschlusselementen ist ein Hakenmaterial vorgesehen, welches an seinen Rändern von einer Schutzschicht aus Nonwoven überdeckt wird. Das Nonwoven erstreckt sich dabei über die Haken des Hakenelementes, wodurch dort nur eine unzureichende Befestigung der Schutzschicht ermöglicht wird. Schließlich besteht auch die Gefahr, dass die Schutzschicht aus Nonwoven durch die Haken unkontrolliert zerfasert wird.

Aus der WO 99/13745 A1 ist ein Hakenmaterial bekannt, welches hakenfreie Ränder aufweist, wobei die hakenfreien Ränder von einem Deckmaterial abgedeckt sind. Die beschriebene Anordnung ist dazu vorgesehen, in Taillenbereich einer Windel angeordnet zu werden, um daran Windelverschlusselemente befestigen zu können, die ein Schlaufenmaterial aufweisen. Es ist nicht beschrieben, die aus der WO 99/13745 A1 bekannte Anordnung abschnittsweise elastisch auszuführen.

Der vorliegenden Erfindung liegt die Aufgabe zugrunde, eine Verbundstoffbahn mit elastischen und unelastischen Bereichen, aus der Windelverschlusselemente ausstanzbar sind, anzugeben, welche kostengünstig und leicht hergestellt werden kann und eine zuverlässige Befestigung eines Hakenmaterials ermöglicht. Des Weiteren sollen auch ein geeignetes Verfahren zur Herstellung einer solchen Verbundstoffbahn und ein im besonderen Maße dafür geeignetes Hakenmaterial angegeben werden.

Gemäß einem ersten Aspekt der Erfindung wird die Aufgabe ausgehend von einer Verbundstoffbahn mit den eingangs beschriebenen Merkmalen dadurch gelöst, dass der Streifen aus Hakenmaterial hakenfreie Ränder aufweist, die jeweils von einem Rand des angrenzenden Nonwovenmaterials überlappt sind. Erfindungsgemäß ist das Hakenmaterial auf verbesserte Weise in die Verbundstoffbahn integriert und zumindest an seinen hakenfreien Rändern innenliegend angeordnet. Die durch die elastisch dehnbaren Folienstreifen gebildeten elastischen Bereiche der Verbundstoffbahn erstrecken sich bis unmittelbar an das Hakenmaterial heran. Auch bei einer wiederholten Beanspruchung der Verbundstoffbahn bzw. eines daraus gebildeten Verschlusselementes kann es nicht zu einem Zerreißen im Bereich des Hakenmaterials kommen, weil dieses an seinen hakenfreien Rändern zumindest durch das Nonwovenmaterial in den Verbund eingebunden ist.

Der vorliegenden Erfindung liegt die Erkenntnis zugrunde, dass bei den aus dem Stand der Technik bekannten Verbundstoffbahnen mit elastischen und unelastischen Bereichen, aus denen Windelverschlusselemente ausstanzbar sind, stets eine zusätzliche Verstärkung im Bereich das Hakenmaterials notwendig ist. Gemäß der vorliegenden Erfindung wird diese Funktion aber von dem Hakenmaterial selbst übernommen, wobei das Hakenmaterial mit seinen hakenfreien Rändern einerseits eine hinreichende Verstärkung der Verbundstoffbahn darstellt und andererseits ein Verhaken mit einer zugeordneten Gegenfläche ermöglicht. Dabei ist zu berücksichtigen, dass auf das Hakenmaterial und insbesondere die Befestigung des Hakenmaterials in der Verbundstoffbahn bei der Benutzung erhebliche Kräfte wirken.

Die Breite der hakenfreien Ränder ist so zu wählen, dass eine sichere Befestigung des angrenzenden Nonwovenmaterials möglich ist. Die Breite jedes hakenfreien Randes kann beispielsweise zwischen 2 mm und 30 mm, vorzugsweise zwischen 8 mm und 20 mm liegen.

Das die Folienstreifen an der zweiten Seite der Verbundstoffbahn abdeckende Nonwovenmaterial liegt zweckmäßigerweise in Form von voneinander beabstandeten Nonwovenstreifen vor, wobei Haken des Hakenmaterials, vorzugsweise sämtliche Haken des Hakenmaterials, zwischen den Nonwovenstreifen angeordnet sind. Im Bereich des Hakenmaterials können die Ränder der Nonwovenstreifen genau entlang den Rändern der jeweils darunter angeordneten elastisch dehnbaren Folienstreifen. Dies kann beispielsweise dadurch erreicht werden, dass die elastisch dehnbaren Folienstreifen als Abschnitte eines entsprechenden Vorlaminates bereitgestellt werden. Ausgehend von diesem gemeinsamen Rand können die elastisch dehnbaren Folienstreifen sowie die darauf angeordneten Folienstreifen entweder eine gleiche oder eine unterschiedliche Breite aufweisen. Bei einer unterschiedlichen Breite ergibt sich dann ein Bereich, in dem die Nonwovenstreifen direkt auf der flächigen Nonwovenbahn aufliegen.

Des Weiteren ist es möglich, dass das Nonwovenmaterial im Bereich des Hakenmaterials über die darunter angeordneten elastisch dehnbaren Folienstreifen hinaus geht, d. h. seitlich über die elastisch dehnbaren Folienstreifen vorsteht. Dies ist insbesondere möglich, wenn die Folienstreifen sowie das Nonwovenmaterial in Streifen separat zugeführt und dann gleichzeitig oder unmittelbar nacheinander kaschiert werden, so dass kein Vorlaminat bereitgestellt werden muss. Mit einer solchen Ausgestaltung ist insbesondere möglich, dass die hakenfreien Räder des Hakenmaterials nur von dem Nonwovenmaterial und nicht von den elastisch dehnbaren Folienstreifen überlappt werden. Die Ränder der elastisch dehnbaren Folienstreifen sind im Rahmen einer solchen Ausgestaltung dann seitlich der hakenfreien Ränder des Hakenmaterials angeordnet, so dass ein besonders gleichmäßiger Übergang erreicht werden kann, insbesondere wenn die Dicke des Hakenmaterials an seinen Rändern in etwa der Dicke der elastisch dehnbaren Folienstreifen entspricht.

Gemäß einer bevorzugten Ausgestaltung der Erfindung ist das Hakenmaterial unmittelbar auf der flächigen Nonwovenbahn befestigt. Auch die elastisch dehnbaren Folienstreifen können - außerhalb des Überlappungsbereiches mit den hakenfreien Rändern des Hakenmaterials - direkt auf der Nonwovenbahn befestigt sein.

Für die Befestigung kommen grundsätzlich unterschiedliche Möglichkeiten in Betracht. Die Befestigung kann beispielsweise durch Klebstoff erfolgen, der wahlweise vollflächig oder nur in Bereichen aufgetragen werden kann. Je nach Art der Verklebung und abhängig von den Fügeparametern kann eine streifenförmige Verklebung ausreichend sein, um das Hakenmaterial sicher zu befestigen. Durch eine streifenförmige Verklebung kann grundsätzlich Klebstoff eingespart werden, wodurch sich eine Kostenreduzierung ergibt. Dabei ist auch zu berücksichtigen, dass geeignete Klebstoffe nicht nur relativ teuer sind, sondern auch die Elastizität beeinträchtigen können. Dieser negative Einfluss des Klebstoffes wird durch einen streifenförmigen Auftrag unterhalb der Folienstreifen reduziert. Vorteilhaft ist insbesondere ein streifenförmiger Klebstoffauftrag parallel zu der Erstreckung des elastisch dehnbaren Folienstreifen.

Die elastisch dehnbaren Folienstreifen besteht aus einem geeigneten thermoplastischen Elastomer, wobei insbesondere ein Polymer aus der Gruppe der Styrol-Butadien-Styrol-Block-Copolymere (SBS), Styrol-Isopren-Styrol-Block-Copolymere (SIS), Styrol-Ethen-Buten-Styrol-Block-Copolymer (SEBS), elastischen Polyethylen-Copolymere, elastische Polypropylen-Copolymere, elastischen Polyurethan-Copolymere, elastische Polyamid-Copolymere, oder einer Mischung dieser Polymere geeignet ist. Neben einer Verwendung von Monofolien können auch coextrudierte Folien eingesetzt werde, wobei auch coextrudierte Folien mit mehreren identischen Schichten geeignet sind. Besonders geeignet sind Folienstreifen mit einer Dicke zwischen 10 und 130 µm.

Das die elastischen Streifen an der zweiten Außenseite abdeckende Nonwovenmaterial sowie die an der gegenüberliegenden Seite der Verbundstoffbahn angeordnete Nonwovenbahn sind üblicherweise selbst nicht elastisch, aber im ausreichenden Maße dehnbar, um die elastischen Bereiche der Verbundstoffbahn bilden zu können. Die notwendige Dehnbarkeit kann aber auch durch eine Aktivierung der Verbundstoffbahn durch ein erstmaliges Dehnen erfolgen, bei dem die Nonwovenschichten auch zu einem Teil zerstört, d. h. zerrissen werden.

Das Nonwoven verleiht der Verbundstoffbahn eine weiche textile Haptik. Je nach Ausgestaltung der Verbundstoffbahn muss das Nonwoven jedoch auch eine gewisse Festigkeit aufweisen. Dies ist insbesondere bei der zuvor beschriebenen Ausgestaltung der Fall, bei der die elastisch dehnbaren Folienstreifen seitlich der Ränder des Hakenmaterials enden oder auf Stoß an die Ränder des Hakenmaterials angrenzen. Das Nonwoven dient dann dazu im Bereich des Übergangs zwischen den elastisch dehnbaren Streifen und dem Hakenmaterial ein Zerreißen zu vermeiden.

Gegenstand der Erfindung ist auch ein Verfahren zur Herstellung einer Verbundstoffbahn mit elastischen und unelastischen Bereichen, aus der Windelverschlusselemente abtrennbar, insbesondere ausstanzbar sind, wobei eine Nonwovenbahn zugeführt wird, wobei ein Hakenmaterial in Streifenform zugeführt wird, welches hakenfreie Ränder aufweist oder an dem hakenfreie Räder gebildet werden, wobei das Hakenmaterial mit einer den Haken gegenüberliegenden, in der Regel ebenen Rückseite auf der Nonwovenbahn befestigt wird und wobei danach elastisch dehnbare Folienstreifen und Nonwovenstreifen derart zugeführt und an der Nonwovenbahn befestigt werden, dass die Folienstreifen zwischen der Nonwovenbahn und den Folienstreifen angeordnet werden und dass die hakenfreien Ränder des Hakenmaterials zumindest teilweise von den Rändern zugeordneter Folienstreifen und/oder Nonwovenstreifen überlappt werden.

Mit dem beschriebenen Verfahren ist die Herstellung der zuvor erläuterten Verbundstoffbahn mit einfachen Mitteln möglich. Wie bereits zuvor im Zusammenhang mit der Verbundstoffbahn selbst erläutert, könnten die Folienstreifen vor dem Verbinden mit der Nonwovenbahn bereits mit jeweils einem zugeordneten Nonwovenstreifen verbunden sein. Insbesondere können die Folienstreifen und die damit verbundenen Nonwovenstreifen als Abschnitte eines entsprechenden Vorlaminates bereitgestellt werden.

Für die Verbindung der einzelnen Elemente der Verbundstoffbahn ergeben sich im Rahmen der Erfindung verschiedenen Möglichkeiten. So kann beispielsweise das Hakenmaterial in Streifenform an einer den Haken gegenüberliegenden Rückseite vollflächig oder alternativ auch nur Abschnittsweise mit der Nonwovenbahn verklebt werden. Mit einer vollflächigen Verklebung ist im Rahmen der Erfindung gemeint, dass der Klebstoff vollflächig auf die Rückseite des Hakenmaterials und/oder die zugeordnete Fläche der Nonwovenbahn aufgebracht wird. Durch die Struktur der Nonwovenbahn können aber Poren oder dergleichen verleiben, an denen lokal keine Verklebung erfolgt.

Bei einer abschnittsweisen Verklebung können insbesondere in Längsrichtung des streifenförmigen Hakenmaterials verlaufende Klebstoffstreifen vorgesehen sein, die sowohl gerade als auch wellenförmig ausgebildet sein können. Bei einer wellenförmigen Verklebung ergibt sich der Vorteil, dass auch die äußeren Ränder stets in einem gewissen Maße verklebt sind.

Des Weiteren kann eine Verbindung der einzelnen Elemente auch durch Ultraschalltechnologie, also ein Ultraschallschweißen erfolgen. Auch ein mechanisches und/oder thermisches Verbinden kommt als weitere Alternative in Betracht.

Wenn die Folienstreifen zusammen mit den Nonwovenstreifen als Abschnitte eines entsprechenden Vorlaminates bereitgestellt werden, müssen bei der Herstellung das Hakenmaterial in Streifenform an der Nonwovenbahn sowie die Abschnitte des Vorlaminates an der Nonwovenbahn und in dem überlappenden Bereich an dem Hakenmaterial befestigt werden. Da der Überlappungsbereich relativ klein ist, muss dort in besonderem Maße auf eine zuverlässige Verbindung geachtet werden. An sämtlichen Verbindungsstellen sind die verschiedenen beschriebenen Verfahren, nämlich ein vollflächiges oder ein abschnittsweise vorgesehenes Verkleben sowie eine Verbindung durch Ultraschalltechnik denkbar. Die Befestigung des Hakenmaterials an der Nonwovenbahn mittels Ultraschalltechnik kann jedoch schwierig sein, weil dabei die Gefahr besteht, dass die freien Haken durch die Beaufschlagung mit Ultraschall zerstört werden. Dieser Nachteil ergibt sich aber nicht, wenn das Ultraschallschweißen nur an den hakenfreien Rändern des Hakenmaterials erfolgt. Grundsätzlich ist auch eine Kombination von Verkleben und Ultraschallschweißen nicht ausgeschlossen.

Ein weiterer Aspekt der vorliegenden Erfindung, dem eine eigenständige erfinderische Bedeutung zukommt, betrifft die Bildung eines Hakenmaterials. Dieses Hakenmaterial ist in besonderem Maße für die zuvor beschriebene Verbundstoffbahn geeignet, grundsätzlich aber auch in anderen Bereichen einsetzbar. Zur Bildung des Hakenmaterials wird eine Polymerschmelze von einer Breitschlitzdüse in einen Spalt zwischen einer Walze und einem abschnittsweise entlang der Oberfläche der Walze geführten Band gegeben, wobei die Walze und/oder das Band eine für die Bildung von Haken vorgesehene Strukturierung aufweisen. Je nach Ausgestaltung des Verfahrens können entweder vollständige Haken mit Stielen und endseitigen Köpfen oder zunächst nur die Stiele der Haken gebildet werden, wobei dann die Köpfe nachträglich erzeugt werden.

Unter Haken werden im Rahmen der Erfindung Elemente verstanden, die dazu geeignet sind, sich in einem zugeordneten Material, beispielsweise einem mit freien Schlaufen gewirkten Textil oder einem Schlaufen aufweisenden Nonwoven, zu verhaken. Die Haken können dabei sowohl an den Stielen umgebogene Enden oder auch pilzförmige Verbreiterungen als Köpfe aufweisen.

Da eine zunächst noch flüssige Polymerschmelze aus der Schlitzdüse auf den Spalt gegeben wird, kann die Ausformung der Haken des Hakenmaterials sehr frei variiert werden. So kann beispielsweise die zur Bildung der Haken vorgesehene Strukturierung an Abschnitten des Bandes bzw. der Walze unterbrochen sein, um dort keine Haken zu bilden. Insbesondere kann die Strukturierung in Querrichtung und/oder Umfangsrichtung unterbrochen sein, wobei dann durch eine ebene Oberfläche hakenfreie Bereiche erzeugt werden.

Neben der Erzeugung von Bereichen mit Haken sowie hakenfreien Bereichen kann bei dem beschriebenen Verfahren auch durch eine entsprechende Konturierung des Bandes und/oder der Walze der Walzenspalt und damit die lokale Dicke des erzeugten Hakenmaterials eingestellt werden. Insbesondere kann in hakenfreien Bereichen durch Vorsprünge des Bandes und/oder der Walze die Dicke des Walzenspaltes reduziert werden, so dass in den für die Bildung der Haken strukturierten Bereichen Trägerabschnitte unterhalb der Haken gebildet werden, die gegenüber den hakenfreien Bereichen eine erhöhte Dicke aufweisen. Eine solche stufenförmige Ausgestaltung ist insbesondere dann von Vorteil, wenn an den hakenfreien Bereichen eine Materialüberlappung wie bei der zuvor beschriebenen Verbundstoffbahn vorgesehen ist. Die Konturierung kann auch durch einen konturierten Extrusions-Düsenspalt erzeugt oder zumindest unterstützt werden. Auch wenn die Schmelze nach der Extrusion noch flüssig und verformbar ist, kann durch eine solche Ausformung mittels des konturierten Extrusions-Düsenspaltes bereichsweise unterschiedlich viel Schmelzmasse zur Verfügung gestellt werden, um dann eine bestimmte Konturierung mittels des Bandes und/oder der Walze zu erleichtern.

Wenn gemäß einer ersten Variante zur Bildung des Hakenmaterials ein glattes auch als Sleeve bezeichnetes Band und eine strukturierte Walze (Chill-Roll) eingesetzt werden, werden die Stiele der Haken durch die strukturierte, üblicherweise gekühlte Walze (Chill-Roll) erzeugt.

Im Rahmen der Strukturierung werden Vertiefungen bzw. Kavitäten zur Ausformung der Stiele der Haken in der Walze gebildet. Die Walzenoberfläche kann dazu beispielsweise mit einem Ätzverfahren oder durch Einsatz eines Lasers bearbeitet werden. Die Anzahl und Verteilung der Kavitäten kann variabel an den jeweiligen Anwendungsfall des zu bildenden Hakenbandes angepasst werden.

Das Band kann beispielsweise vollständig nahtlos durch einen galvanischen Prozess erzeugt werden. Ein solches Band, welches abhängig von der Anwendung auch als Glättband bezeichnet wird, wird üblicherweise zwischen zwei temperierten Walzen gespannt, von denen mindestens eine angetrieben werden kann. Das gespannte Band wird üblicherweise durch einen verfahrbaren Walzenstuhl gegen die strukturierte, gekühlte Walze gepresst. Durch das Anpressen des Bandes in einem üblicherweise variabel einstellbaren Umschlingungswinkel wird erreicht, dass die zunächst noch flüssige Polymerschmelze die Vertiefungen bzw. Kavitäten der strukturierten Walze ausfüllt. Auf eine zusätzliche Entlüftung kann dabei in der Regel verzichtet werden, wobei auch eine mehrschichtige Ausgestaltung der Walze zur Entlüftung nicht notwendig ist. Durch den Kontakt mit dem umlaufenden Band während der Ausbildung der Stiele der Haken kann die Abkühlung besser kontrolliert werden, wodurch eine Fertigung auch bei einer sehr hohen Produktionsgeschwindigkeit möglich ist. Durch den Einsatz des beschriebenen Bandes kann auch eine besonders gleichmäßige, glatte und hochwertige Rückseite des Hakenmaterials erzeugt werden.

Wie bereits zuvor erläutert, kann die Strukturierung in Längsrichtung, Querrichtung oder auch in einer beliebigen Richtung unterbrochen sein, um hakenfreie Bereiche zu erzeugen.

Gemäß einer zweiten Variante zur Bildung des Hakenmaterials werden eine glatte Walze und ein strukturiertes bzw. perforiertes Band eingesetzt. Bei einem perforierten Band ist unter allen Umständen eine ausreichende Entlüftung der einzelnen Löcher zur Erzeugung der Stiele der Haken gewährleistet. Durch die Dicke des Bandes kann auch die Länge der auszuformenden Stiele eingestellt werden. Alternativ kann die Länge der Stiele auch durch den Anpressdruck des Bandes an die Walze eingestellt werden, wobei dann bei einem reduzierten Anpressdruck die Löcher der Perforation nur teilweise ausgefüllt werden. Durch eine Variation des Anpressdruckes kann dabei ohne einen Austausch des Bandes die Höhe der Stiele der Haken verändert werden.

Neben einer im Wesentlichen punktförmigen Perforation oder Strukturierung der Walze bzw. des Bandes können an dem Band oder der Walze auch flächig ausgedehntere Vorsprünge vorgesehen sein, um den Spalt zwischen der Walze und dem Band bereichsweise zu variieren. So ist gemäß einer weiteren Variante zur Bildung des Hakenmaterials vorgesehen, dass sowohl die Walze als auch das Band mit einer Strukturierung und/oder mit Vorsprüngen versehen sind. So kann beispielsweise das Band mit einer abschnittsweisen Perforation versehen sein, während die Kühlwalze mit Vorsprüngen versehen ist, die sich beispielsweise linienförmig in Umfangsrichtung oder in Querrichtung erstrecken können. Durch eine derartige Ausgestaltung kann die Dickenverteilung des Hakenmaterials eingestellt werden. Insbesondere ist es möglich, dass unterhalb der Haken gebildete Trägerabschnitte eine größere Dicke als hakenfreie Bereiche aufweisen. Auf vorteilhafte Weise stehen dann die Haken weit nach außen vor, wobei durch die reduzierte Dicke an den hakenfreien Bereichen eine Materialersparnis möglich ist.

Je nach dem, wie die Haken gebildet werden, kann aber auch ausgehend von einem Material mit zunächst gleichbleibender Dicke durch das Bilden der Haken eine Einschnürung daraus resultieren, dass das Material der Haken nach außen gezogen wird. Wenn dann bei der Herstellung keine Polymermasse von den Seiten nachfließt, ergibt sich ausgehend von einer zunächst einheitlichen Dicke über die gesamte Breite an dem Trägerabschnitt unterhalb der Haken eine Einschnürung, aus der die Haken jedoch weiterhin im ausreichenden Maße herausstehen. Durch die mögliche Reduzierung der Dicke an den hakenfreien Bereichen kann dieser ungewünschte, nachteilige Effekt in einem gewissen Maße ausgeglichen werden.

Durch eine Strukturierung der Walze und des Bandes ist es auch möglich, an beiden Seiten des Hakenmaterials Haken zu bilden.

Wenn das Band mit einer Perforation versehen ist, kann gemäß einer bevorzugten Weiterbildung vorgesehen sein, dass die Polymerschmelze zunächst durch die Perforation hindurchgedrückt wird und dann unmittelbar an der innen liegenden Seite des umlaufenden Bandes durch eine weitere Einrichtung zur Bildung von Köpfen umgebogen bzw. flachgedrückt wird. Innerhalb des umlaufenden Bandes kann dazu beispielsweise eine weitere Walze oder auch ein zusätzliches Band vorgesehen sein.

Alternativ kann die an die Stiele anschließende Kopfstruktur auch in einem nachträglichen Prozessschritt erzeugt werde, wobei die zuvor gebildeten Stiele thermisch und/oder mechanisch verformt werde. Die mechanische Umformung der Stielenden zur Bildung der Köpfe geschieht bevorzugt in einem Walzenspalt, in dem mindestens eine Walze angetrieben und die den Stielenden zugewandte Walze beheizbar ist. Zusätzlich kann das Hakenband vorab auf eine Umwärmtemperatur vorgewärmt werde, wozu insbesondere ein Infrarotstrahler geeignet ist. Darüber hinaus ist auch die Bildung der Kopfstruktur durch Einsatz eine Ultraschalltechnik möglich, wobei die Kopfstrukturen in einem Spalt einer Walze und einer Sonotrode durch einen definierten Anpressdruck unter Ultraschall gebildet werden.

Bezüglich der Handhabung der zuvor beschriebenen Verbundstoffbahn kann es vorteilhaft sein die Stiele zunächst ohne Köpfe oder gekrümmte Enden zu bilden, wobei dann diese zur Verhakung vorgesehenen Ausformungen auch erst nach der Bildung der gesamten Verbundstoffbahn erzeugt werden. Ohne die zur Verhakung vorgesehenen Ausformungen in Form von Köpfen oder gekrümmten Enden kann die Verbundstoffbahn nämlich problemlos auf- und wieder abgewickelt werden, ohne dass die zunächst noch gerade verlaufenden Stiele sich verhaken können. Erst wenn die Verbundstoffbahn zur weiteren Verarbeitung, also insbesondere zur Windelherstellung abgewickelt wird, werden die Köpfe oder gekrümmten Enden geformt, wozu beispielsweise eine Druckbeaufschlagung in einem erwärmten Walzenspalt vorgesehen sein kann.

Wie zuvor beschrieben, können auch an beiden Seiten des Hakenmaterials Haken oder Stiele gebildet werden. Insbesondere ist es möglich an der einen Seite des Hakenmaterials Haken und an der Seite lediglich Stiele auszubilden. Wenn ein solches Hakenmaterial für die Herstellung der zuvor beschriebenen Verbundstoffbahn eingesetzt wird, kann das Hakenmaterial mit seinen gerade verlaufenden Stielen auf die durchgehende Nonwovenbahn aufgelegt werden, welche die erste Außenseite der Verbundstoffbahn bildet. Die Stiele durchstechen dann diese Kunststoffbahn und stehen als Abstandshalter vor. Die gegenüberliegenden Haken liegen dann wie zuvor beschrieben frei an der gegenüberliegenden Seite zwischen den Streifen des dort vorgesehenen Nonwovenmaterials. Wenn eine solche Verbundstoffbahn mehrlagig aufgewickelt wird, liegen bei aufeinanderfolgenden Lagen die Stiele und Haken übereinander, wobei durch die Länge der Stiele die darunter liegende Lage der Verbundstoffbahn auf Abstand gehalten wird, wodurch auch im aufgewickelten Zustand vermieden wird, dass die Haken die einzelnen Lagen in unerwünschter Weise miteinander verbinden.

Das Hakenmaterial kann je nach Anforderung und Einsatzbereich aus unterschiedlichen Materialien sowohl einschichtig als auch mehrschichtig aufgebaut sein. Bei einem einschichtigen Aufbau sind insbesondere polyolefinische Materialien wie Polyethylen (PE), Polypropylen (PP), Mischungen der genannten Polymere sowie ein Copolymer von Polyethylen und Polypropylen geeignet. Besonders bevorzugt werden aber steife PP-Typen eingesetzt, die sowohl als Homo- oder als Copolymere vorliegen können. Darüber hinaus sind aber auch andere Polymere für einen einschichtigen oder mehrschichtigen Aufbau geeignet. Vorteilhaft sind insbesondere auch vergleichsweise steife Materialien wie Cycloolefin-Copolymer (COC) Polyester wie beispielsweise Polyethylenterephthalat (PET), Polyamide (PA) wie beispielsweise PA 6 und PA 6,6 oder auch Polymethylmethacrylat (PMMA).

Durch einen mehrschichtigen Aufbau des Hakenbandes können die zum Teil einander entgegenstehenden Anforderungen von z. B. Steifigkeit, Fließfähigkeit während der Herstellung und Kompatibilität zu den im Laminat benachbarten Schichten auf vorteilhafte Weise erfüllt werden. Gemäß einer ersten Variante sind weitgehend typenreine Aufbauten oder zumindest Aufbauten vollständig aus Polyolefin möglich, wodurch insbesondere auch eine Wiederverwertung erleichtert wird. Beispielsweise kann bei einem dreischichtigen Aufbau für alle Schichten Polypropylen (PP) vorgesehen sein, wobei jedoch die Schicht, aus der die Haken gebildet werden sollen, speziell hinsichtlich eines verbesserten Prägeverhaltens mit einem hohen Fließindex (MFI) ausgewählt werden, während die übrigen Schichten gezielt hinsichtlich der erforderlichen Steifigkeit des Hakenbandes optimiert sind.

Neben einem solchen typenreinen Aufbau ist auch ein mehrschichtiger Aufbau aus verschiedenen Materialtypen möglich, wobei in den Außenschichten bevorzugt Materialien auf polyolefinischer Basis, insbesondere PP, eingesetzt werden. Bei einem Aufbau mit zumindest drei Schichten kann eine innenliegende Kernschicht hinsichtlich einer maximalen Steifigkeit ausgewählt sein. Während bei einem fünfschichtigen Aufbau zwischen der Kernschicht und den Außenschichten jeweils eine Haftvermittlerschicht angeordnet wird, kann bei einem dreischichtigen Aufbau eine ausreichende Verbindung der Schichten untereinander durch funktionalisierte bzw. modifizierte Polyolefine erreicht werden.

Die Dicke des Hakenmaterials beträgt an den hakenfreien Rändern sowie unterhalb der Haken typischerweise zwischen 20 und 180 µm. Wenn die hakenfreien Ränder nicht mit einer geringeren Höhe versehen ist, beträgt die Dicke an diesen Rändern sowie die Dicke des unter dem Haken angeordneten Trägerabschnittes besonders bevorzugt zwischen 70 und 140 µm. Dabei ist jedoch zu berücksichtigen, dass durch das bilden der Haken der Trägerabschnitt ausgehend von der angegebenen Dicke im gewissen Maße eingeschnürt sein kann.

Bei einer einschichtigen Ausgestaltung des Hakenmaterials sind sämtliche Abschnitte des Hakenmaterials aus dem gleichen Polymer gebildet. Bei einem mehrschichtigen Aufbau sind dagegen die Haken üblicherweise aus dem Material der entsprechenden Außenschicht gebildet. Wenn dann jedoch die Steifigkeit der Haken für einen bestimmten Anwendungsfall zu gering ist, ist auch eine andere Ausgestaltung möglich, bei der die Haken bei einem mehrschichtigen Aufbau zumindest teilweise aus dem Material einer innen liegenden Schicht gebildet sind. Die kann beispielsweise dadurch erreicht werden, dass bei einem mehrschichtigen Aufbau die Dicke der Außenschicht, an der die Haken erzeugt werden, soweit reduziert wird, dass auch das Material der darunterliegenden Schicht bei einem entsprechenden Prägevorgang wesentlicher Bestandteil der Stiele bzw. Haken wird.

Die Länge der Haken ist üblicherweise deutlich größer als die Dicke eines Trägerabschnittes des Hakenmaterials, auf dem die Haken angeordnet sind. Die Länge der Haken beträgt vorzugsweise das zwei- bis zehnfache, besonders bevorzugt das drei- bis siebenfache der Dicke des Trägerabschnittes unterhalb der Haken.

Gegenstand der Erfindung ist schließlich auch ein Hakenmaterial in Streifenform, insbesondere für die zuvor beschriebene Verbundstoffbahn, wobei das Hakenmaterial auf einem Trägerabschnitt angeordnete Haken sowie seitlich dazu hakenfreie Ränder aufweist. Erfindungsgemäß weisen die hakenfreien Ränder eine gegenüber dem Trägerabschnitt reduzierte Dicke auf, wobei die Dicke an den hakenfreien Rändern vorzugsweise weniger als 80 % der Dicke des Trägerabschnitts beträgt. Die hakenfreie Fläche beträgt bevorzugt zwischen 10 % und 60 % der Gesamtfläche. Bei einem solchen Hakenmaterial ergibt sich der Vorteil, dass die hakenfreien Ränder vergleichsweise dünn sind, so dass es leicht möglich ist, dort ein anderes Material überlappend anzuordnen.

Bezogen auf die zuvor beschriebene Verbundstoffbahn ist eine besonders vorteilhafte Fertigung möglich. Dabei ist zu berücksichtigen, dass das Hakenmaterial unterhalb der Haken eine ausreichende Dicke aufweisen müssen; um die einzelnen Haken zu halten. In den Randbereichen werden die Zugkräfte aber gleichmäßig verteilt, so dass dort eine geringere Dicke ausreichend ist. Durch die reduzierte Dicke an den hakenfreien Rändern wird zunächst erreicht, dass die einzelnen Haken weiter gegenüber dem an der zweiten Außenseite der Verbundstoffbahn angeordneten Nonwovenmaterial vorsteht. Auch ist die Stufe kleiner, die bei der erfindungsgemäß vorgesehenen Überlappung von den elastisch dehnbaren Folienstreifen überbrückt werden muss. Die Stabilität der gesamten Verbundstoffbahn kann dadurch verbessert werden.

Die vorliegende Erfindung wird im Folgenden anhand von Zeichnungen erläutert, welche lediglich Ausführungsbeispiele darstellen. Es zeigen in einer schematische Darstellung
- **Fig. 1**: einen Teilabschnitt einer Verbundstoffbahn in einem Querschnitt,
- **Fig. 2**: die in der Fig. 1 dargestellten Elemente in einer voneinander getrennten Darstellung,
- **Fig. 3A bis 3C**: alternative Ausgestaltungen der Verbundstoffbahn in einer Ansicht gemäß der Fig. 1,
- **Fig. 4A**: die Anordnung gemäß der Fig. 3, wobei ein größerer Bereich der Verbundstoffbahn dargestellt wird,
- **Fig. 4B**: ein aus der Verbundstoffbahn gemäß der Fig. 4a ausgestanztes Wiederverschlusselement,
- **Fig. 4C**: eine Draufsicht auf eine Verbundstoffbahn mit einem Stanzmuster zum Abtrennen einzelner Verschlusselemente,
- **Fig. 5A**: eine bevorzugte Ausgestaltung eines Hakenmaterials,
- **Fig. 5B**: eine alternative Ausgestaltung des Hakenmaterials,
- **Fig. 6**: eine Anordnung zur Erzeugung eines Hakenmaterials,
- **Fig. 7A bis 7D**: ein Hakenmaterial mit hakenfreien Bereichen in unterschiedlichen Ausgestaltungen,
- **Fig. 8A bis 8D**: ein Hakenmaterial gemäß der Fig. 7A bis 7D mit einer zusätzlichen bereichsweisen Variation der Dicke,
- **Fig. 9A bis 9C**: ein Hakenmaterial mit Haken an beiden Seiten,
- **Fig. 10**: eine in Lagen aufgewickelte Verbundstoffbahn.

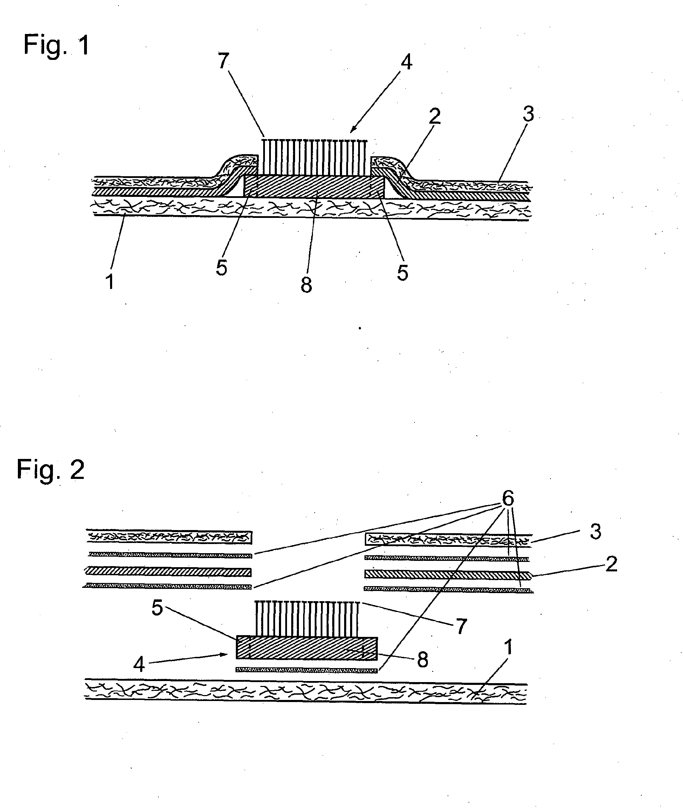

Die Fig. 1 zeigt im Querschnitt einen Ausschnitt aus einer Verbundstoffbahn mit elastischen und unelastischen Bereichen, aus der Windelverschlusselemente ausstanzbar sind. Die Verbundstoffbahn umfasst eine flächige Nonwovenbahn 1, welche eine erste Außenseite der Verbundstoffbahn bildet. Um die elastischen Bereiche der Verbundstoffbahn zu erzeugen, sind parallele zueinander beabstandete, elastische dehnbare Folienstreifen 2 auf der Nonwovenbahn 1 angeordnet, wobei diese Folienstreifen 2 von einem Nonwovenmaterial 3 abgedeckt sind. Das in Form von Nonwovenstreifen angeordnete Nonwovenmaterial 3 deckt die Folienstreifen 2 vollständig ab und bildet bereichsweise eine zweite Außenseite der Verbundstoffbahn.

Zusätzlich ist ein Hakenmaterial 4 unmittelbar auf der Nonwovenbahn 1 befestigt, wobei ein Bereich zwischen den dargestellten benachbarten elastisch dehnbaren Folienstreifen 2 von dem Hakenmaterial 4 überbrückt wird. Während das vergleichsweise steife Hakenmaterial 4 den unelastischen Bereiche der Verbundstoffbahn fixiert, schließen die elastischen Bereiche der Verbundstoffbahn an beiden Seiten des Hakenmaterials 4 an, wobei sowohl die Nonwovenbahn 1 an der ersten Außenseite und das Nonwovenmaterial 3 an der zweiten Außenseite der Verbundstoffbahn dehnbar sind. Die elastische Rückstellung wird dabei durch den elastisch dehnbaren Folienstreifen 2 erreicht. Gegebenenfalls ist vor einer erstmaligen Benutzung ein zumindest abschnittsweise vorgenommenes Recken in Querrichtung zweckmäßig, um eine Aktivierung, das heißt eine leichte Dehnbarkeit der Verbundstoffbahn in den elastischen Bereichen, zu erreichen. Auch bei einer solchen Aktivierung sind die unelastischen Bereiche durch das vergleichsweise starre Hakenmaterial 4 fixiert.

Erfindungsgemäß weist das Hakenmaterial 4 hakenfreie Ränder 5 auf, die jeweils von einem Rand der angrenzenden elastisch dehnbaren Folienstreifen 2 überlappt sind. Die Folienstreifen 2 sind dabei in geeigneter Weise an den hakenfreien Rändern 5 befestigt, so dass die Verbundstoffbahn in dem dargestellten Ausschnitt in Querrichtung ohne Schwachstelle ist. Da das Hakenmaterial 4 in die Verbundstoffbahn integriert ist und auch an den hakenfreien Rändern 5 eine Überlappung mit den Folienstreifen 2 vorgesehen ist, ist eine zusätzliche Versteifung, wie sie aus dem Stand der Technik bekannt ist, nicht erforderlich.

In dem dargestellten Ausführungsbeispiel werden nicht nur die Folienstreifen 2 sondern auch das Nonwovenmaterial 3 und das Hakenmaterial 4 als Streifen zugeführt, die entlang ihrer Längsrichtung eine unveränderte Ausgestaltung aufweisen. Grundsätzlich ist es aber nicht ausgeschlossen, dass das Hakenmaterial 4 entsprechend der Abmessungen einzelner Windelverschlusselemente in Streifenlängsrichtung hakenfreie Bereiche aufweisen.

Die Befestigung des Hakenmaterials 4 an der Nonwovenbahn 1 sowie die Befestigung der elastisch dehnbaren Folienstreifen 2 an der Nonwovenbahn 1 sowie an den hakenfreien Rändern 5 kann auf unterschiedliche Weise erfolgen. Insbesondere kann ein vollflächiges oder abschnittsweises Verkleben mittels Klebstoff 6 oder auch ein Ultraschallschweißen erfolgen. Bei einem lediglich abschnittsweise vorgesehenen Verkleben erfolgt die Verbindung zweckmäßigerweise durch Klebstoffstreifen, die sich entlang der Längsrichtung der beschriebenen streifenförmigen Elemente erstrecken.

In der Fig. 2 sind die einzelnen Bestandteile der Verbundstoffbahn dargestellt, wobei lediglich exemplarisch ein flächiger Auftrag von Klebstoff 6 gezeigt ist. Bei dem Verfahren zur Herstellung der Verbundstoffbahn mit elastischen und unelastischen Bereichen wird zunächst die Nonwovenbahn 1 zugeführt. Nachfolgend wird das Hakenmaterial 4 in Streifenform zugeführt, welches hakenfreie Ränder 5 aufweist. Das Hakenmaterial 4 wird dann mit einer den Haken 7 gegenüberliegenden ebenen Rückseite auf der Nonwovenbahn 1 befestigt, also beispielsweise mit dem Klebstoff 6 verklebt. Nachfolgend werden dann die elastisch dehnbaren Folienstreifen 2 und Nonwovenstreifen des Nonwovenmaterials 3 derart zugeführt und an der Nonwovenbahn 1 befestigt, dass die Folienstreifen 2 zwischen der Nonwovenbahn 1 und dem Nonwovenmaterial 3 angeordnet werden und dass die hakenfreien Ränder 5 des Hakenmaterials 4 zumindest teilsweise von den Rändern zugeordneter Folienstreifen 2 sowie Nonwovenstreifen des Nonwovenmaterials 3 überlappt werden. Dabei ist darauf zu achten, dass die Folienstreifen 2 mit den hakenfreien Rändern 5 verbunden werden, wozu in dem dargestellten Ausführungsbeispiel Klebstoff 6 vorgesehen ist.

Anders als in Fig. 2 dargestellt, können die Folienstreifen 2 und das Nonwovenmaterial 3 bereits vor der Verbindung mit der Nonwovenbahn 1 bzw. zu dem Hakenmaterial 4 miteinander laminiert sein, so dass dann Streifen eines entsprechenden Vorlaminates zugeführt werden.

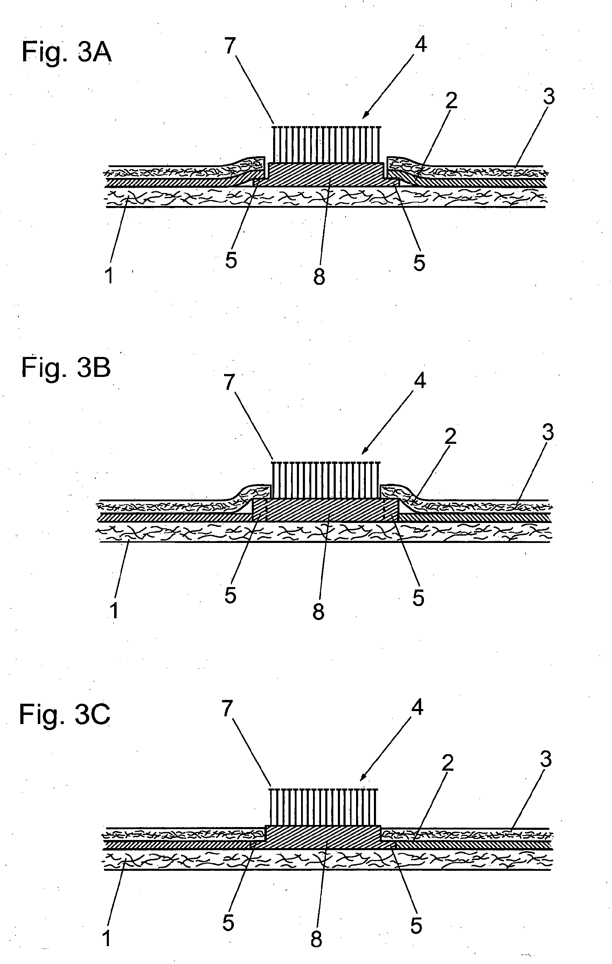

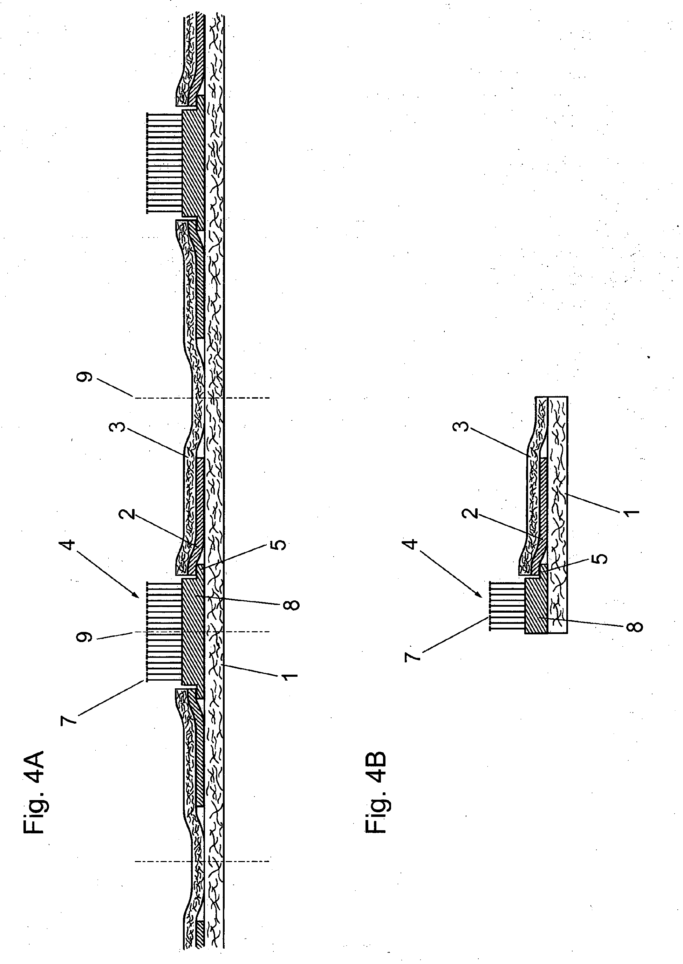

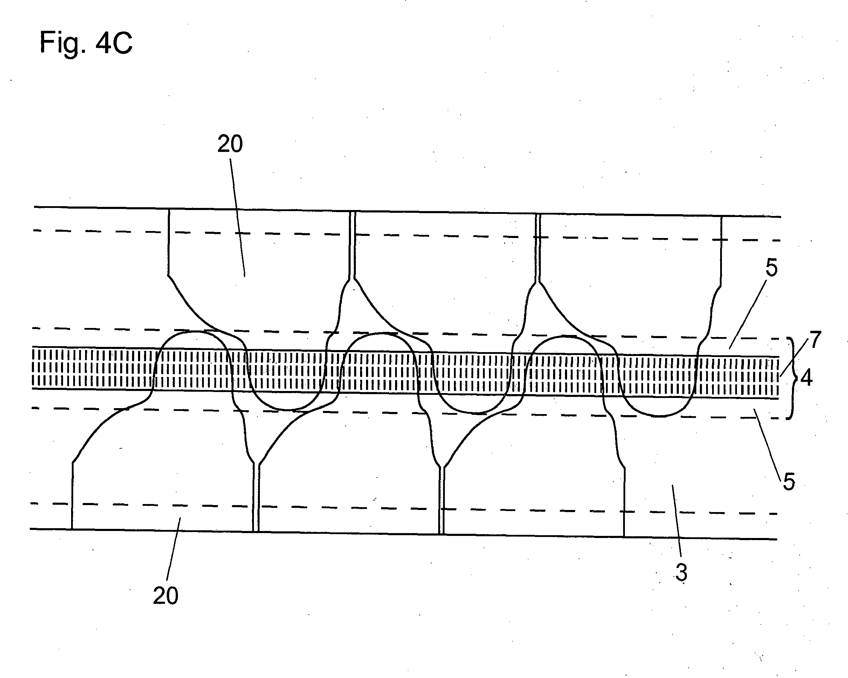

Die Fig. 3A betrifft eine bevorzugte Weiterbildung des beschriebenen Erfindungskonzeptes mit einem gegenüber der Fig. 1 veränderten Hakenmaterial 4. Das in der Fig. 4 im Detail dargestellte Hakenmaterial 4 weist hakenfreie Ränder 5 mit einer reduzierten Dicke auf. Zwischen den hakenfreien Rändern 5 befinden sich Haken 7 auf einem mittleren Abschnitt des Hakenmaterials 4, der als Trägerabschnitt 8 bezeichnet wird. Der Trägerabschnitt 8 muss eine ausreichende Dicke aufweisen, um die einzelnen Haken 7 sicher halten zu können und um eine ausreichende Versteifung der Verbundstoffbahn zu erreichen. Dagegen weisen die hakenfreien Ränder 5 gegenüber dem Trägerabschnitt 8 eine reduzierte Dicke auf, wodurch sich bereits eine erhebliche Materialeinsparung ergibt. Zusätzlich ergeben sich aber auch hinsichtlich der Geometrie verschiedene Vorteile. So ist unmittelbar zu erkennen, dass die Folienstreifen 2 und das Nonwovenmaterial 3 weniger stark verformt sind, so dass die Verbundfestigkeit in Querrichtung insgesamt verbessert werden kann. Zusätzlich ist auch erkennbar, dass die Funktion der Haken 7 nicht durch die Folienstreifen 2 und das Nonwovenmaterial 3 beeinträchtigt ist, während bei der Ausgestaltung gemäß der Fig. 1 zumindest eine gewisse Beeinträchtigung der außenliegenden Haken 7 nicht ausgeschlossen werden kann. Zumindest liegt bei einer ansonsten übereinstimmenden Ausgestaltung eine größere freie Länge vor, die zur Verbindung mit einem komplementären Material zur Verfügung steht.

Die Fig. 3B und 3C zeigen alternative Ausgestaltungen, bei denen die elastisch dehnbaren Folienstreifen 2 seitlich vor dem Hakenmaterial 4 enden, so dass die hakenfreien Ränder 5 nur von den Nonwovenstreifen des Nonwovenmaterials 3 abgedeckt sind. Im Rahmen einer solchen Ausgestaltung müssen die Nonwovenbahn 1 und das Nowovenmaterial 3 eine ausreichende Festigkeit aufweisen, um an dem Übergang zu dem Hakenmaterial 4 ein Zerreißen zu vermeiden. Als Vorteil ergibt sich aber ein besonders flacher Aufbau, wobei die elastisch dehnbaren Folienstreifen 2 vollkommen eben auf der durchgehenden Nonwovenbahn 1 angeordnet sind. Während gemäß der Fig. 3B ein Hakenmaterial 4 gemäß der Fig. 1 eingesetzt wird, zeigt die Fig. 3C eine Ausgestaltung, bei der das Hakenmaterial der Ausführung gemäß der Fig. 3A entspricht. Bei der Ausgestaltung gemäß der Fig. 3C kann insbesondere die Dicke der hakenfreien Ränder 5 an die Dicke der elastisch dehnbaren Folienstreifen 2 angepasst werden, so dass sich an den Nonwovenstreifen des Nonwovenmaterials 3 nur eine geringe oder gar keine Stufe ergibt.

Die Fig. 1 bis 3C zeigen lediglich ein Ausschnitt der Verbundstoffbahn, aus der einzelne Windelverschlusselemente ausstanzbar sind. Die Verbundstoffbahn kann im Bereich des Hakenmaterials 4 durchtrennt, so dass dann das geschnittene Hakenmaterial 4 an einem äußeren Ende des Windelverschlusselementes liegt. An der gegenüberliegenden Seite wird das Windelverschlusselement dann an einem Windelkörper befestigt, beispielsweise verklebt oder durch Ultraschall verschweißt. Durch die Verklebung erfolgt eine Stabilisierung und Versteifung des Windelverschlusselementes an dem Ende, welches dem Hakenmaterial 4 gegenüberliegt.

Im Rahmen der Erfindung ist es grundsätzlich möglich, dass die Folienstreifen 2 die gleiche Breite aufweisen, wie das darauf angeordnete Nonwovenmaterial 3 in Form von Nonwovenstreifen. Es ist aber auch möglich, dass sich die Folienstreifen 2 nur über einen Teil des Nonwovenmaterials 3 erstrecken, so dass dann zwei Folienstreifen 2 mit einem Nonwovenstreifen des Nonwovenmaterials 3 kombiniert werden. Eine entsprechende Ausgestaltung ist in der Fig. 4A dargestellt, wobei auch die Schnittlinien 9 zur Bildung einzelner Windelverschlusselemente (Fig. 4B) dargestellt sind. Die Windelverschlusselemente können auf der Verbundstoffbahn ausgestanzt werden. Grundsätzlich kommen aber auch ein Zerschneiden der Verbundstoffbahn oder eine andere Auftrennung in Betracht. Anstelle von geraden Schnittlinien, wie sie in der Fig. 4B der Einfachheit halber angedeutet sind, kann auch ein wellenförmiges Schneiden bzw. Stanzen erfolgen, so dass dann schmaler werdende Enden erzeugt werden, welche jeweils das Hakenmaterial mit den Haken über ihre gesamte Breite aufweisen.

So zeigt die Fig. 4C ein mögliches Schnitt- bzw. Stanzmuster zur Erzeugung einzelner Verschlusselemente 20. In der Ausgestaltung gemäß der Fig. 4C weist jedes Verschlusselement 20 den mit Haken 7 versehenen Bereich des Hakenmaterials 4 über seine gesamte Breite auf. Zusätzlich wird ein freies Ende des Verschlusselementes 20 neben den Haken 7 von einem Abschnitt der Verbundstoffbahn gebildet, welcher einen der beiden hakenfreien Ränder 5 des Hakenmaterials 4 umfasst. Es ergibt sich der Vorteil, dass auch dieser von einem Benutzer an einer Windel oder einem vergleichbaren Produkt zu ergreifende Abschnitt durch den hakenfreien Rand 5 des Hakenmaterials 7 steif und nicht dehnbar ist, wobei durch den Wegfall der Haken 7 und die Oberfläche aus dem Nonwovenmaterial 3 eine einfache und komfortable Handhabung möglich ist.

Durch das in der Fig. 4C dargestellte Schnitt- bzw. Stanzmuster mit im Bereich des Hakenmaterials 4 ineinander greifenden Verschlusselementen 20 kann der Verschnitt minimiert werden.

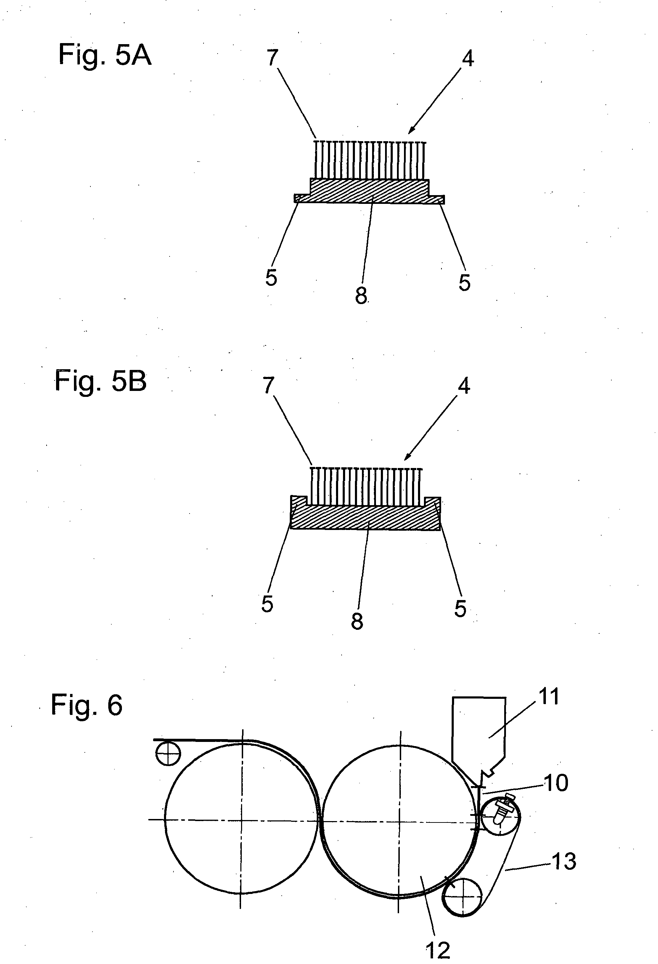

Die hakenfreien Ränder 5 können auf unterschiedliche Weise gebildet werden. Beispielsweise kann ausgehend von einem vollständig besetzten Hakenmaterial vorgesehen sein, dass an den Rändern Haken abgetrennt werden, um die hakenfreien Ränder 5 zu bilden. Darüber hinaus ist es aber auch möglich, ausgehend von einem vollständig mit Haken besetzten Hakenmaterial, Haken durch Druck und Temperatur aufzuschmelzen und so zu entfernen. Dabei kann auch durch eine entsprechende Kraftbeaufschlagung die Dicke der hakenfreien Ränder 5 reduziert werden, um zu einer Ausgestaltung gemäß der Fig. 5A zu gelangen. Je nach Anforderungen gemäß dem jeweils vorgesehenen Anwendungszweck, kann es aber auch von Vorteil sein, wenn die hakenfreien Ränder 5 gegenüber dem unterhalb der Haken 7 angeordneten Trägerabschnitt 8 eine erhöhte Dicke aufweisen (Fig. 5B). Die freie Länge der Haken 7 ist zwar reduziert, jedoch weist ein solches Hakenmaterial hier aufgrund der reduzierten Dicke des Trägerabschnittes 8 eine verbesserte Flexibilität auf und wird auch bei der Benutzung weniger steif. Je nach Ausgestaltung ist es aufgrund der Flexibilität beispielsweise auch möglich, dass die Haken 7 durch eine Verformung des Trägerabschnittes 8 in ein textiles Schlaufenelement als Gegenmaterial eingedrückt werden.

Die beschriebenen Maßnahmen zur Erzeugung der hakenfreien Ränder 5 sind aber vergleichsweise aufwendig, weshalb es bevorzugt ist, die hakenfreien Ränder 5 direkt bei der Herstellung des Hakenmaterials 4 zu erzeugen.

So kann gemäß der Fig. 6 vorgesehen sein, dass zur Bildung des Hakenmaterials 4 eine Polymerschmelze 10 aus einer Schlitzdüse 11 in den Spalt zwischen einer gekühlten Walze 12 (Chill-Roll) und einem abschnittsweise entlang der Oberfläche der Walze 12 geführten Band 13 gegeben wird, wobei die Walze 12 und/oder das Band 13 eine für die Bildung von Haken 7 vorgesehene Strukturierung aufweisen. Die Strukturierung kann in Form von Vertiefungen bzw. Kavitäten oder insbesondere bei dem Band 13 in Form einer Perforation vorgesehen sein. Das in sich geschlossene Band 13 wird auch als Glättband oder Sleeve bezeichnet.

Die Strukturierung zur Bildung von Haken ist im Wesentlichen punktförmig, um Stiele der Haken 7 bilden zu können. Direkt bei der Herstellung können mit einer Anordnung gemäß der Fig. 6 hakenfreie Bereiche dadurch gebildet werden, dass die beschriebene Strukturierung unterbrochen ist, wobei dort die Walze 12 bzw. das Band 13 eine glatte Oberfläche aufweisen.

Neben einer feinen Strukturierung zur Bildung einzelner Haken 7 können an der Walze und dem Glättband auch Vorsprünge oder Nuten vorgesehen sein, die sich über einen größeren Bereich der Oberfläche erstrecken, wobei dadurch die Dicke des Walzenspaltes bereichsweise unterschiedlich eingestellt werden kann. Wenn der Verlauf solcher Vorsprünge oder Nuten an die Strukturierung zur Bildung der Haken 7 angepasst ist, können beispielsweise hakenfreie Bereiche mit einer reduzierten Dicke erzeugt werden. So ist es insbesondere möglich, in den für die Bildung der Haken 7 strukturierten Bereichen Tragabschnitte 8 unterhalb der Haken 7 zu bilden, die gegenüber den hakenfreien Bereichen eine erhöhte Dicke aufweisen.

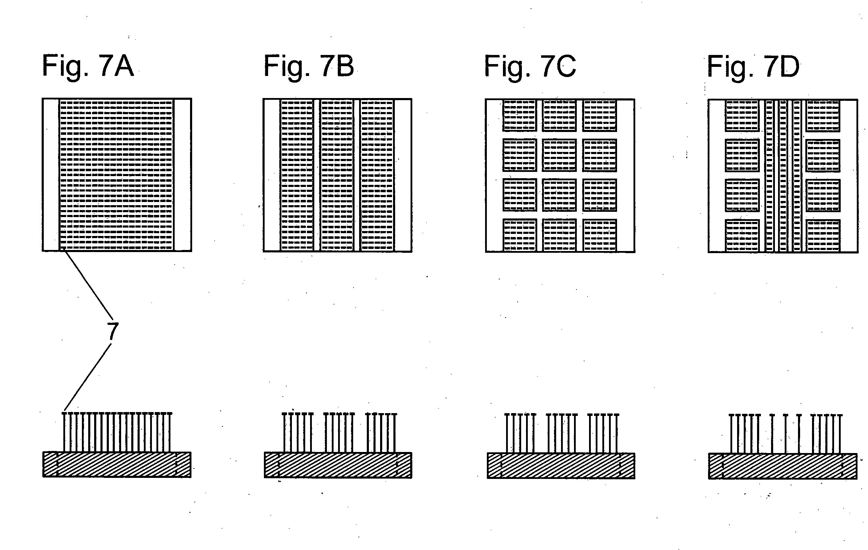

Mit der beschriebenen Methode kann ein Hakenmaterial in Streifenform erzeugt werden, welches im besonderen Maße für die zuvor beschriebene Verbundstoffbahn geeignet ist. Das Hakenmaterial kann aber auch für andere Anwendungszwecke vorgesehen sein. Die Fig. 7A bis 7D zeigen exemplarisch unterschiedliche Ausgestaltungen des Hakenmäterials 4 mit hakenfreien Bereichen in einer Draufsicht sowie in einem Querschnitt. Bei der Fig. 7A wird ein streifenförmiges Hakenmaterial erzeugt, welches hakenfreie Ränder 5 aufweist. Es liegt also eine Übereinstimmung mit dem Hakenmaterial 4 gemäß der Fig. 1 vor. Gemäß der Fig. 7B können in Längsrichtung des Hakenmaterials mehrere mit Haken 7 versehene Bereiche sowie hakenfreie Bereiche angeordnet werden.

Fig. 7C zeigt, dass zusätzlich auch in Längsrichtung des streifenförmigen Hakenmaterials 4 Unterbrechungen vorgesehen sein können, wobei schließlich die Fig. 7D eine Kombination von in Längsrichtung durchgehenden sowie in Längsrichtung unterbrochenen Bereichen mit Haken 7 zeigt.

Wie zuvor erläutert, können zusätzlich zu der Strukturierung zur Bildung der Haken 7 auch Vorsprünge sowie Nuten bzw. Rücksprünge an der Walze 12 sowie dem Band 13 vorgesehen sein, um den Spalt zwischen der Walze 12 und dem Band 13 zu verändern.

Bezogen auf das Hakenmaterial 4 handelt es sich bei der Strukturierung zur Bildung der Haken 7 um eine Mikrostrukturierung, während die Vorsprünge und Rücksprünge die makroskopische Struktur des Hakenmaterials 4 bestimmen. Insbesondere können die Vor- und Rücksprünge auf die Strukturierung zur Bildung der Haken 7 abgestimmt sein.

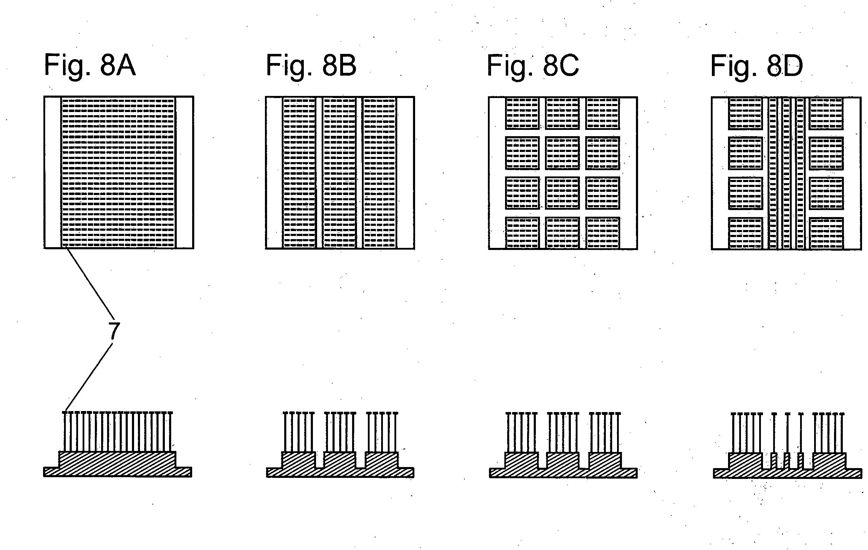

Die Fig. 8A bis 8D zeigen Hakenmaterial 4 entsprechend der Fig. 7A bis 7D, wobei jedoch die hakenfreien Bereiche eine geringe Dicke aufweisen, als Trägerabschnitte 8 die unterhalb der Haken 7 gebildet sind.

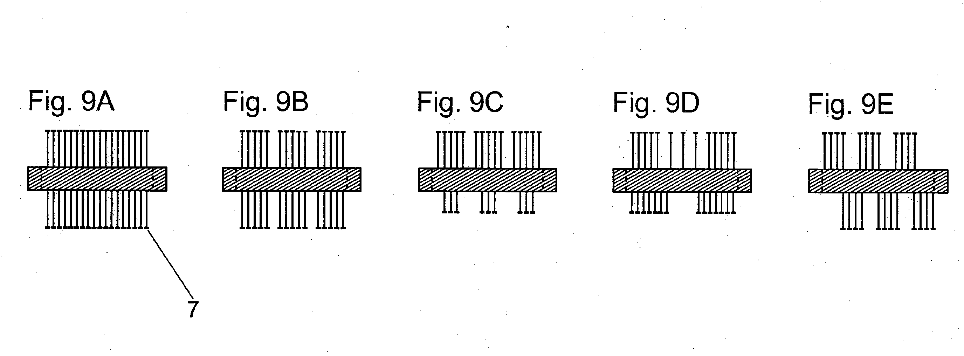

Wenn sowohl die Walze 12 als auch das Band 13 mit einer Strukturierung zur Bildung von Haken 7 versehen sind, kann ein Hakenmaterial 4 mit Haken 7 an beiden Seiten erzeugt werden. Dieses Hakenmaterial 4 ist dann nicht für die zuvor beschriebene Verbundstoffbahn vorgesehen, kann aber beispielsweise als Verbindungselement eingesetzt werden. Die Fig. 9A zeigt ein Hakenmaterial, welches entsprechend der Fig. 7 ausgestaltet ist, jedoch an seinen beiden Seiten Haken 7 aufweisen. Die Fig. 9B entspricht der Ausgestaltung gemäß der Fig. 7B, wobei aber auch hier Haken 7 an beiden Seiten vorgesehen sind. Selbstverständlich können auch bei der Anordnung von Haken 7 an beiden Seiten des Hakenmaterials 4 Unterbrechungen in Längsrichtung vorgesehen sein (vgl. Fig. 7C). In den Fig. 9C bis 9E ist dargestellt, dass die Haken 7 an beiden Seiten des Hakenmaterials 4 unterschiedlich ausgestaltet sein können, wobei die Haken 7 eine unterschiedliche Länge aufweisen können. Darüber hinaus kann auch die Position und die Ausdehnung der mit Haken 7 versehenen Bereiche unterschiedlich sein.

Das Hakenmaterial 4 besteht vorzugsweise aus Polyolefin, beispielsweise aus Polyethylen, Polypropylen, Copolymeren von PE und PP oder Mischungen der genannten Materialien. Das Hakenmaterial kann einschichtig ausgebildet sein, wobei aber auch der mehrschichtige Aufbau, insbesondere ein Aufbau mit bis zu fünf Schichten, möglich ist. Bei einem mehrschichtigen Aufbau besteht bevorzugt zumindest eine Schicht aus einem Polyolefin. Bei einem mehrschichtigen Aufbau ist es auch möglich, eine innenliegende Schicht oder bei der Anordnung von Haken 7 an lediglich einer Seite des Hakenmaterials 4 eine den Haken 7 gegenüberliegende Außenschicht mit einer erhöhten Festigkeit zu versehen, um das Material möglichst stabil auszugestalten. Gerade wenn eine solche Schicht sich in den hakenfreien Bereichen streckt, kann auch bei einer sehr geringen Dicke eine hohe Stabilität erreicht werden.

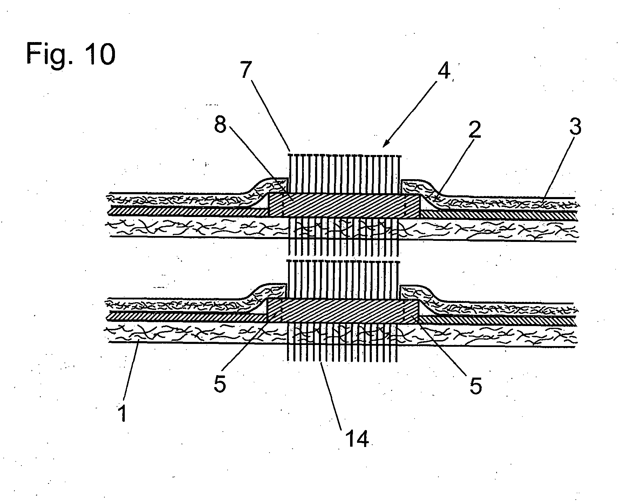

Die Fig. 10 zeigt exemplarisch einen möglichen Anwendungsfall, bei dem die Ausbildung und Ausformungen an beiden Seiten des Hakenmaterials 4 zweckmäßig ist. Gemäß dem Ausführungsbeispiel der Fig. 10 wird ein Hakenmaterial 4 eingesetzt, welches an einer Seite die zuvor beschriebenen Haken 7 aufweist. An der gegenüberliegenden Seite sind Stiele 14 angeordnet, die im Wesentlichen wie die Haken gefertigt werden, wobei jedoch keine Köpfe oder umgebogene Enden gebildet werden.

Während die Haken 7 gemäß der Fig. 1 angeordnet sind, sind die Stiele 14 in Richtung der flächigen Nonwovenbahn 1 ausgerichtet und durchstechen diese, wodurch auch eine mechanische Verzahnung zwischen dem Hakenmaterial 4 und der Nonwovenbahn 1 erreicht wird. Besonders vorteilhaft ist aber, dass die Stiele 14 bei dem Aufwickeln der Verbundstoffbahn in mehreren Lagen als Abstandshalter dienen und in einem gewissen Maße die Haken 7 der angrenzenden Lage auf Abstand halten können.

Bei der weiteren Verarbeitung der Verbundstoffbahn können dann die Stiele beispielsweise mit der Nonwovenbahn 1 durch Druck und Temperatur verschmolzen werden, wodurch auch noch eine zusätzliche Versteifung erreicht werden kann.

## Patentansprüche

1. Verbundstoffbahn mit elastischen und unelastischen Bereichen, aus der Windelverschlusselemente abtrennbar bzw. ausstanzbar sind, umfassend eine Nonwovenbahn (1), welche eine erste Außenseite der Verbundstoffbahn bildet, zueinander beabstandete, elastisch dehnbare Folienstreifen (2), die auf der Nonwovenbahn (1) angeordnet sind, ein Nonwovenmaterial (3), welches an einer zweiten Außenseite der Verbundstoffbahn die Folienstreifen (2) abdeckt, und zumindest einen nichtelastischen Streifen aus einem Hakenmaterial (4), der einen Bereich zwischen zwei benachbarten elastischen dehnbaren Folienstreifen (2) überbrückt, **dadurch gekennzeichnet, dass** der Streifen aus Hakenmaterial (4) hakenfreie Ränder (5) aufweist, die jeweils von einem Rand des angrenzenden Nonwovenmaterials (3) überlappt sind.

2. Verbundstoffbahn nach Anspruch 1, **dadurch gekennzeichnet, dass** das Nonwovenmaterial (3) an der zweiten Seite der Verbundstoffbahn in Form von voneinander beabstandeten Nonwovenstreifen angeordnet ist, wobei Haken (7) des Hakenmaterials (4) zwischen den Nonwovenstreifen angeordnet sind.

3. Verbundstoffbahn nach Anspruch 1 oder 2, **dadurch gekennzeichnet, dass** das Hakenmaterial (4) unmittelbar auf der Nonwovenbahn (1) befestigt ist.

4. Verbundstoffbahn nach einem der Ansprüche 1 bis 3, **dadurch gekennzeichnet, dass** die elastisch dehnbaren Folienstreifen (2) an der Nonwovenbahn (1) sowie an jeweils einem ihrer Ränder an einem der hakenfreien Ränder (5) des Hakenmaterials (4) befestigt sind.

5. Verbundstoffbahn nach einem der Ansprüche 1 bis 4, **dadurch gekennzeichnet, dass** das Hakenmaterial (4) aus Polyolefin gebildet ist.

6. Verbundstoffbahn nach einem der Ansprüche 1 bis 5, **dadurch gekennzeichnet, dass** das Hakenmaterial (4) einen mehrschichtigen Aufbau aufweist.

7. Verfahren zur Herstellung einer Verbundstoffbahn mit elastischen und unelastischen Bereichen, aus der Windelverschlusselemente abtrennbar, insbesondere ausstanzbar sind,
wobei eine Nonwovenbahn (1) zugeführt wird,
wobei ein Hakenmaterial (4) in Streifenform zugeführt wird, welches hakenfreie Ränder (5) aufweist,
wobei das Hakenmaterial (4) mit einer den Haken (7) gegenüberliegenden Rückseite auf der Nonwovenbahn (1) befestigt wird,
wobei danach elastisch dehnbare Folienstreifen (2) und Nonwovenstreifen derart zugeführt und an der Nonwovenbahn (1) befestigt werden, dass die Folienstreifen (2) zwischen der Nonwovenbahn (1) und den Nonwovenstreifen angeordnet werden und dass die hakenfreien Ränder (5) des Hakenmaterials (4) zumindest teilweise von den Rändern zugeordneter Folienstreifen (2) und/oder Nonwovenstreifen überlappt werden.

8. Verfahren nach Anspruch 7, wobei die Folienstreifen (2) vor dem Verbinden mit der Nonwovenbahn (1) jeweils bereits mit einem zugeordneten Nonwovenstreifen verbunden sind.

9. Verfahren nach Anspruch 8, wobei die Folienstreifen (2) und die damit verbundenen Nonwovenstreifen als Abschnitte eines entsprechenden Vorlaminates bereitgestellt werden.

10. Verfahren nach einem der Ansprüche 7 bis 9, wobei das Hakenmaterial (4) an einer den Haken (7) gegenüberliegenden Rückseite vollflächig mit der Nonwovenbahn (1) verklebt wird.

11. Verfahren nach einem der Ansprüche 7 bis 9, wobei das Hakenmaterial (4) an einer den Haken (7) gegenüberliegenden Rückseite mit in Längsrichtung verlaufenden Klebstoffstreifen mit der Nonwovenbahn (1) verklebt wird.

12. Verfahren nach einem der Ansprüche 7 bis 9, wobei das Hakenmaterial (4) mittels Ultraschallschweißen mit der Nonwovenbahn (1) verbunden wird.

13. Verfahren nach einem der Ansprüche 7 bis 12, wobei zur Bildung des Hakenmaterials (4) eine Polymerschmelze (10) von einer Schlitzdüse (9) in einen Spalt zwischen einer Walze (12) und einem abschnittsweise entlang der Oberfläche der Walze (12) geführten Band (13) gegeben wird und wobei die Walze (12) und/oder das Band (13) eine für die Bildung von Haken (7) vorgesehene Strukturierung aufweist.

14. Verfahren nach Anspruch 13, wobei die Struktur in Querrichtung und/oder Umfangsrichtung des Bandes (13) bzw. der Walze (12) unterbrochen ist, um hakenfreie Bereiche zu bilden.

15. Verfahren nach Anspruch 14, wobei in den hakenfreien Bereichen durch Vorsprünge des Bandes (13) und/oder der Walze (12) die Dicke des Walzenspaltes reduziert ist, so dass in den für die Bildung der Haken (7) strukturierten Bereichen Trägerabschnitte (8) unterhalb der Haken (7) gebildet werden, die gegenüber den hakenfreien Bereichen eine erhöhte Dicke aufweisen.

16. Hakenmaterial (4) in Streifenform, insbesondere für eine Verbundstoffbahn nach einem der Ansprüche 1 bis 6, mit hakenfreien Rändern (5) und mit Haken (7), die zwischen den hakenfreien Rändern (5) auf einem Trägerabschnitt (8) angeordnet sind, **dadurch gekennzeichnet, dass** die hakenfreien Ränder (5) eine gegenüber dem Trägerabschnitt (8) reduzierte Dicke aufweisen.

17. Hakenmaterial (4) nach Anspruch 16, **dadurch gekennzeichnet, dass** die Dicke an den hakenfreien Rändern (5) weniger als 80 % der Dicke des Trägerabschnitts (8) beträgt.

18. Hakenmaterial (4) nach Anspruch 16 oder 17, **dadurch gekennzeichnet, dass** die hakenfreie Fläche zwischen 10 % und 60 % der Gesamtfläche beträgt.

## Claims

1. A composite material web with elastic and inelastic regions from which nappy closure elements can be separated or punched, comprising a nonwoven web (1) which forms a first outer side of the composite material web, elastically stretchable film strips (2) spaced apart from one another which are disposed on the nonwoven web (1), a nonwoven material (3) which covers the film strip (2) on a second outer side of the composite material web and at least one non-elastic strip of a hook material (4) which bridges a region between two neighbouring elastic stretchable film strips (2), **characterized in that** the strip of hook material (4) has hook-free edges (5), which are each overlapped by an edge of the adjacent nonwoven material (3).

2. The composite material web according to claim 1, **characterized in that** the nonwoven material (3) is disposed on the second side of the composite material web in the form of nonwoven strips spaced apart from one another, wherein hooks (7) of the hook material (4) are disposed between the nonwoven strips.

3. The composite material web according to claim 1 or 2, **characterized in that** the hook material (4) is fastened directly on the nonwoven web (1).

4. The composite material web according to any one of claims 1 to 3, **characterized in that** the elastically stretchable film strips (2) are fastened on the nonwoven web (1) as well as at respectively one of their edges on one of the hook-free edges (5) of the hook material (4).

5. The composite material web according to any one of claims 1 to 4, **characterized in that** the hook material (4) is formed from polyolefin.

6. The composite material web according to any one of claims 1 to 5, **characterized in that** the hook material (4) has a multilayer structure.

7. A method for manufacturing a composite material web with elastic and inelastic regions from which nappy closure elements can be separated, in particular punched out,
wherein a nonwoven web (1) is supplied,
wherein a hook material (4) in strip form is supplied which has hook-free edges (5),
wherein the hook material (4) is fastened with a rear side opposite the hooks (7) on the nonwoven web (1),
wherein thereafter elastically stretchable film strips (2) and nonwoven strips are supplied and fastened to the nonwoven web (1) in such a manner that the film strips (2) are disposed between the nonwoven web (1) and the nonwoven strip and that the hook-free edges (5) of the hook material (4) are overlapped at least partially by the edges of associated film strips (2) and/or nonwoven strips.

8. The method according to claim 7, wherein the film strips (2) are each already joined to an associated nonwoven strip before joining to the nonwoven web (1).

9. The method according to claim 8, wherein the film strips (2) and the nonwoven strips joined thereto are provided as sections of a corresponding prelaminate.

10. The method according to any one of claims 7 to 9, wherein the hook material (4) on a rear side opposite the hooks (7) is extensively adhesively bonded to the nonwoven web (1).

11. The method according to any one of claims 7 to 9, wherein the hook material (4) on a rear side opposite the hooks (7) is adhesively bonded to the nonwoven web (1) with adhesive strips running in the longitudinal direction.

12. The method according to any one of claims 7 to 9, wherein the hook material (4) is joined to the nonwoven web (1) by means of ultrasound welding.

13. The method according to any one of claims 7 to 12, wherein to form the hook material (4) a polymer melt (10) is fed from a slotted nozzle (9) into a gap between a roller (12) and a band (13) guided in sections along the surface of the roller (12) and wherein the roller (12) and/or the band (13) has a structuring provided to form hooks (7).

14. The method according to claim 13, wherein the structure is interrupted in the transverse direction and/or circumferential direction of the band (13) or the roller (12) to form hook-free regions.

15. The method according to claim 14, wherein in the hook-free regions the thickness of the roller gap is reduced by projections of the band (13) and/or the roller (12) so that in the structured regions to form the hooks (7) carrier sections (8) are formed underneath the hooks (7) which have an increased thickness compared with the hook-free regions.

16. A hook material (4) in strip form, in particular for a composite material web according to any one of claims 1 to 6, having hook-free edges (5) and having hooks (7) which are disposed between the hook-free edges (5) on a carrier section (8), **characterized in that** the hook-free edges (5) have a reduced thickness compared with the carrier section (8).

17. The hook material (4) according to claim 16, **characterized in that** the thickness at the hook-free edges (5) is less than 80 % of the thickness of the carrier section (8).

18. The hook material (14) according to claim 16 or 17, **characterized in that** the hook-free area is between 10 % and 60 % of the total area.

## Revendications

1. Bande de matériau composite comportant des zones élastiques et non élastiques, dans laquelle des éléments de fermeture de couches peuvent être découpés ou estampés, comprenant une nappe de non-tissé (1) qui constitue une première face extérieure de la bande de matériau composite, des bandes de film (2) espacées mutuellement et extensibles élastiquement qui sont disposées sur la nappe de non-tissé (1), un matériau non tissé (3) qui couvre les bandes de film (2) sur une seconde face extérieure de la bande de matériau composite et au moins une bande non élastique de matériau accrochant (4) qui chevauche une zone entre deux bandes de film (2) extensibles et élastiques, **caractérisée en ce que** la bande de matériau accrochant (4) présente des bords sans crochets (5) qui sont respectivement chevauchés par un bord du matériau non tissé adjacent (3).

2. Nappe de matériau composite selon la revendication 1, **caractérisée en ce que** le matériau non tissé (3) est disposé sur la seconde face de la bande de matériau composite sous forme de bandes non tissées espacées mutuellement, les crochets (7) du matériau accrochant (4) étant disposés entre les bandes non tissées.

3. Nappe de matériau composite selon la revendication 1 ou 2, **caractérisée en ce que** le matériau accrochant (4) est fixé directement sur la nappe de non-tissé (1).

4. Nappe de matériau composite selon une des revendications 1 à 3, **caractérisée en ce que** les bandes de film (2) extensibles élastiquement sont fixées à la nappe de non-tissé (1) et respectivement à un de ses bords au niveau d'un des bords sans crochets (5) du matériau accrochant (4).

5. Nappe de matériau composite selon une des revendications 1 à 4, **caractérisée en ce que** le matériau accrochant (4) est constitué de polyoléfine.

6. Nappe de matériau composite selon une des revendications 1 à 5, **caractérisée en ce que** le matériau accrochant (4) présente une structure multicouche.

7. Procédé de fabrication d'une bande de matériau composite comportant des zones élastiques et non élastiques, dans lequel des éléments de fermeture de couches peuvent être découpés, en particulier estampés, dans lequel
une nappe de non-tissé (1) est acheminée,
un matériau accrochant (4) qui présente des bords sans crochets (5) est acheminé sous forme de bande,
le matériau accrochant (4) est fixé à une face arrière opposée aux crochets (7) sur la bande non tissée (1),
des bandes de film (2) extensibles élastiquement et des bandes non tissées sont ensuite acheminées et fixées à la nappe de non-tissé (1) de manière à ce que les bandes de film (2) soient disposées entre la nappe de non-tissé (1) et la bande non tissée et que les bords sans crochets (5) du matériau accrochant (4) soient chevauchés du moins partiellement par les bandes de film (2) associées aux bords et/ou les bandes non tissées.

8. Procédé selon la revendication 7, dans lequel, avant d'être reliées avec la nappe de non-tissé (1), les bandes de film (2) sont déjà reliées à une nappe de non-tissé associée.

9. Procédé selon la revendication 8, dans lequel les bandes de film (2) et les bandes non tissées sont fournies sous forme de sections d'un prélaminé correspondant.

10. Procédé selon une des revendications 7 à 9, dans lequel le matériau accrochant (4) est collé sur une face arrière opposée aux crochets (7) sur toute la surface de la nappe de non-tissé (1).

11. Procédé selon une des revendications 7 à 9, dans lequel le matériau accrochant (4) est collé au niveau d'une face arrière opposée aux crochets (7) par un cordon de colle s'étendant dans le sens longitudinal à la nappe de non-tissé (1).

12. Procédé selon une des revendications 7 à 9, dans lequel le matériau accrochant (4) est relié par soudage aux ultrasons à la nappe de non-tissé (1).

13. Procédé selon une des revendications 7 à 12, dans lequel, pour former le matériau accrochant (4), une coulée de polymère (10) est dispensée par une buse fendue (9) dans un intervalle entre un cylindre (12) et une bande (13) guidée par sections le long de la surface du cylindre (12) et le cylindre (12) et/ou la bande (13) présente une structuration prévue pour former des crochets (7).

14. Procédé selon la revendication 13, dans lequel la structure est interrompue dans le sens transversal et/ou le sens circonférentiel de la bande (13) ou du cylindre (12) pour former des zones sans crochets.

15. Procédé selon la revendication 14, dans lequel, dans les zones sans crochets, l'épaisseur de l'intervalle entre les cylindres est réduite par des saillies de la bande (13) et/ou du cylindre (12) de sorte que, dans les zones structurées pour former les crochets (7), des sections porteuses (8) qui sont plus épaisses que les zones sans crochets sont constituées sous les crochets (7).

16. Matériau accrochant (4) sous forme de bande, en particulier pour Nappe de matériau composite selon une des revendications 1 à 6, comportant des bords sans crochets (5) et des crochets (7) qui sont disposés entre les bords sans crochets (5) sur une section porteuse (8), **caractérisé en ce que** les bords sans crochets (5) sont moins épais que la section porteuse (8).

17. Matériau accrochant (4) selon la revendication 16, **caractérisé en ce que** l'épaisseur des bords sans crochets (5) représente moins de 80 % de l'épaisseur de la section porteuse (8).

18. Matériau accrochant (4) selon la revendication 16 ou 17, **caractérisé en ce que** la surface sans crochets représente 10 % à 60 % de la surface totale.
